# EUROPÄISCHE PATENTSCHRIFT

(11) **EP 1 768 449 B1**
(45) Veröffentlichungstag und Bekanntmachung des Hinweises auf die Patenterteilung: **19.08.2009**
(21) Anmeldenummer: 06120912.8
(22) Anmeldetag: 19.09.2006
(51) Int. Cl.: H04R 25/00

(54) **Verfahren zur Einstellung einer Hörvorrichtung anhand biometrischer Daten und entsprechende Hörvorrichtung**
Device and method of adjusting a hearing apparatus based on biometric data
Méthode pour ajustage d'un dispositif auditif basé sur des données biométriques

(30) Priorität: 27.09.2005 DE 102005046168
(43) Veröffentlichungstag der Anmeldung: 28.03.2007
(73) Patentinhaber: Siemens Audiologische Technik GmbH, 91058 Erlangen (DE)
(72) Erfinder: Rass, Uwe, 90480 Nürnberg (DE)
(74) Vertreter: Maier, Daniel Oliver

(56) Entgegenhaltungen:
- WO-A-01/37914
- JP-A- 2002 143 103
- US-A1- 2005 192 514
- US-B1- 6 230 047

## Beschreibung

Die vorliegende Erfindung betrifft ein Verfahren zur **Einstellung eines** Hörgeräts. Darüber hinaus betrifft die vorliegende Erfindung **ein entsprechendes Hörgerät** mit einer Sensoreinrichtung zum Erfassen biometrischer Daten des Nutzers **des Hörgeräts.**

Für die automatische Einstellung eines Hörgeräts kann es von Vorteil sein, wenn das Hörgerät von dem Benutzer eine Rückmeldung erhält. Wenn der Benutzer in bestimmten Hörsituationen an dem Lautstärkesteller oder dem Programmumschalter des Hörgeräts Einstellungen vornimmt, können diese Betätigungen vom Hörgerät registriert und weiterverarbeitet werden. So können derartige Lautstärkeeinstellungen beispielsweise gelernt und für eine ähnliche Hörsituation wieder verwendet werden. Dieses automatische Lernen findet nach rein objektiven Gesichtspunkten statt und berücksichtigt nicht subjektive Gemütsschwankungen. So kann beispielsweise die Lautstärkeeinstellung des Hörgeräts an einem Tag für den Hörgeräteträger angenehm sein, während er diese Lautstärkeeinstellung am nächsten Tag trotz des gleichen Eingangschallpegels als unangenehm empfindet.

Aus der Druckschrift WO 01/37914 A1 ist ein Verfahren und ein Gerät zum Abspielen von Musik in Echtzeitsynchronität mit dem Herzschlag bekannt. Dabei wird der Pulsschlag eines Patienten detektiert und in ein elektrisches Signal gewandelt. Das elektrische Signal wird dazu verwendet, ein Musikstück zu gestalten. Ein Kopfhörer oder Lautsprecher gibt dann das Musikstück an den Patienten aus.

Weiterhin beschreibt die Patentschrift US 6,230,047 B1 ein Gerät zum Erzeugen von pulsgetriggerten Musikrhythmen. Der Aufbau des Geräts ist ähnlich dem des oben genannten Beispiels.

Das Dokument US 2005/0192514 A1 offenbart ein audiologisches Behandlungssystem und ein entsprechendes Verfahren zum Behandeln neurologischer Fehlfunktionen mit Schalltherapie. Das System besteht aus einem örtlichen Gerät und einem entfernt gelegenen Gerät, die beide miteinander über ein Netzwerk in Verbindung stehen. Das örtliche Gerät dient zum Ermitteln und Behandeln der audiologischen Fehlfunktion. Mit einem Ohrstück können biometrische Daten wie Temperatur, Puls, Schweißabsonderung und dergleichen ermittelt werden.

Die Aufgabe der vorliegenden Erfindung besteht somit darin, die automatischen Einstellmöglichkeiten für eine Hörvorrichtung bzw. ein Hörgerät zu verbessern.

Der Erfindung liegt der Gedanke zu Grunde, für das automatische Einstellen der Hörvorrichtung auch biometrische Daten, die den Gemütszustand des Nutzers widerspiegeln, zu verwenden.

In diesem Zusammenhang ist aus der Druckschrift JP 2002-143103 A bekannt, mit Hilfe eines Hörgeräts biometrische Daten zu messen. Diese biometrischen oder Körperdaten, wie Temperatur, Blutdruck und Puls, werden an ein externes Gerät weitergegeben. Das Hörgerät dient hier also zur reinen Datenerfassung. Die Körperdaten dienen jedoch nicht zur Einstellung des Hörgeräts.

Die oben genannte Aufgabe wird erfindungsgemäß gelöst, durch ein Verfahren gemäß Anspruch 1.

Darüber hinaus wird erfindungsgemäß bereitgestellt ein Hörgerät gemäß Anspruch 7.

In vorteilhafter Weise ist es somit **möglich, ein** Hörgerät nach dem subjektiven Gefühlszustand des Nutzers automatisch einzustellen. Damit kann der Nutzer ein eventuelles Nachregulieren vermeiden, das durch rein individuelle Gefühle bedingt ist. Für das automatische Einstellen des Hörgeräts werden also nicht nur Messgrößen verwendet, die die akustische Hörsituation betreffen, sondern auch Größen, die den subjektiven Gefühlszustand widerspiegeln.

Vorzugsweise wird unmittelbar vor oder während des Erfassens der biometrischen Daten dem Nutzer ein Schall aus **dem Hörgerät** dargeboten. Die biometrischen Daten geben dann Auskunft über den Zustand des Nutzers. Wenn der Nutzer beispielsweise den dargebotenen Schall als Stress empfindet, steigt sein Puls.

In speziellen Ausführungsformen können als biometrische Daten der Hautwiderstand, die Temperatur, der Blutzucker, der Blutsauerstoffgehalt und/oder der Puls des Nutzers gemessen werden. Mit einer entsprechend hohen Vielzahl an Messwerten kann **das Hörgerät** sehr differenziert eingestellt werden.

In Abhängigkeit der biometrischen Daten kann ein Frequenzgang, die Lautstärke und/oder eine Störgeräuschunterdrückung **des Hörgeräts** angepasst werden. Damit lässt sich berücksichtigen, wie der Nutzer einen verstärkten Schall als angenehm oder unangenehm empfindet.

Darüber hinaus kann in Abhängigkeit des Pulses des Nutzers ein Rhythmus eines dargebotenen oder darzubietenden Musikstücks angepasst werden. Damit kann der Nutzer allmählich in einen bestimmten Zustand, beispielsweise einen Ruhezustand, gebracht werden.

Ferner kann es von Vorteil sein, in Abhängigkeit der biometrischen Daten in **dem Hörgerät** mindestens ein Steuersignal zu erzeugen und dieses an ein externes Gerät zu senden. Damit lässt sich der über **das Hörgerät** ermittelte Gefühlszustand auch zur Steuerung externer Geräte verwenden.

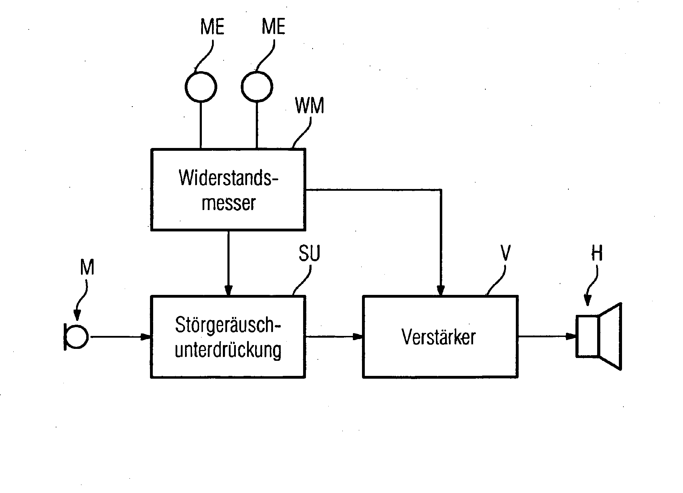

Die vorliegende Erfindung wird nun anhand der beigefügten Zeichnung näher erläutert, die eine Prinzipskizze eines erfindungsgemäßen Hörgeräts darstellt.

Die nachfolgend näher geschilderten Ausführungsbeispiele stellen bevorzugte Ausführungsformen der vorliegenden Erfindung dar.

Der im Ohr oder auch am Kopf getragene Teil des Hörgeräts (HdO, IdO oder Ohrpassstück eines HdOs) enthält Messeinrichtungen für den Hautwiderstand, die Temperatur, den Blutzucker, den Sauerstoffgehalt im Blut und/oder den Puls. In dem in der FIG dargestellten Beispiel wird der Hautwiderstand über zwei Metallelektroden ME oder über leitfähige Kunststoffe an der Gehäuseaußenseite gemessen. Ein Widerstandsmesser WM ermittelt den Hautwiderstand aus den Messsignalen. Das resultierende Widerstandssignal dient nun zur Steuerung klassischer Signalverarbeitungseinrichtungen des Hörgeräts. Im vorliegenden Beispiel wird die Störgeräuschunterdrückungseinheit SU und die Verstärkereinheit V mit diesem Widerstandsmesssignal gesteuert.

Im Folgenden werden vier mögliche Anwendungen geschildert, in denen ein Biofeedback eines Hörgeräts genutzt werden kann:
a) Überwachung des Stress-Zustands aus den Parametern von Bio-Signalen und akustischem Signal:
   Je nach Zustand des Nutzers können Parameter des Hörgeräts, wie Frequenzgang, Lautstärke, Störgeräuschunterdrückung und dergleichen angepasst werden. Das Ziel ist, dadurch den Stress zu reduzieren. Stress kann beispielsweise in geräuscherfüllter Umgebung entstehen, wenn man versucht, sich auf einen Geräuschpartner zu konzentrieren. Beispielsweise wird der Hautwiderstand wird als Maß für den Stress, dem der Hörgeräteträger ausgesetzt ist, verwendet. In dem Hörgerät wird dann in Abhängigkeit des Hautwiderstands eine Störgeräuschunterdrückung des über ein Mikrofon M aufgenommenen Eingangsschalls durchgeführt. Ebenso wird stressabhängig, also abhängig vom Hautwiderstand, der Verstärker V und damit die Lautstärke an einem Hörer H des Hörgeräts reguliert.
b) Medizinischer Trainingsmodus:
   Die Parameter der Bio-Signale werden auf ein akustisches Signal abgebildet, das im Hörgerät wiedergegeben wird. Zum Beispiel wird der Puls durch einen Rhythmus eines Musikstücks für den Hörgeräteträger hörbar dargestellt. Die Aufgabe des Benutzers ist es nun, den Rhythmus zu verlangsamen, um sich beispielsweise zu entspannen. Eine derartige Entspannungsübung kann für Herzinfarktpatienten von Interesse sein. Für Sportler kann auf diese Weise der Puls überwacht werden, wobei das Signal beispielsweise eine Musik ist, deren Abspielgeschwindigkeit verändert wird. Dabei können die Musikstücke entweder im Hörgerät selbst oder in einer externen Einheit (z. B. MP3-Player) gespeichert werden. Des Weiteren kann der oben genannte, sich durch den Puls verändernde Rhythmus auch für ein Herzkohärenztraining benutzt werden. Der Herzschlag synchronisiert sich dabei auf den dargebotenen Rhythmus.
c) Unterstützung der Anpassung:
   Durch Einbeziehen der Parameter der detektierten Bio-Signale lassen sich die Aussagen des Patienten während der Anpassung von Hörgeräten präzisieren. Die Bio-Signale bieten die Möglichkeit, psychoakustische Größen objektiv zu messen (z. B. die Lautheit, die Schärfe, Höranstrengung, etc.).
d) Steuerung von externen Geräten:
   Die Parameter der Bio-Signale eventuell kombiniert mit akustischen Parametern werden benutzt, um externe Geräte zu steuern. Das Hörgerät sendet hierzu die detektierten Signal-Parameter drahtlos zu einer Empfangseinheit (z. B. ein
   Notebook oder PDA). Die Signale werden in der Empfangseinheit ausgewertet und in Aktionen umgesetzt. Beispiele dieser Anwendung sind: Bedienung eines Telefons, Fernbedienung für TV, Rollstuhlsteuerung, Steuerung von künstlichen, elektrisch betriebenen Prothesen, etc.

## Patentansprüche

1. Verfahren zum Einstellen eines Hörgeräts, das einen **von einem Mikrofon (M) des Hörgeräts aufgenommenen** Eingangsschall zu einem Ausgangsschall verarbeitet,
**gekennzeichnet durch**
- Erfassen von biometrischen Daten (WM) des Nutzers des Hörgeräts und
- Einstellen mindestens eines Parameters (SU, V) des Hörgeräts, so dass das Verarbeiten des Eingangsschalls **durch** das Hörgerät in Abhängigkeit von den erfassten biometrischen Daten erfolgt.

2. Verfahren nach Anspruch 1, wobei unmittelbar vor oder während des Erfassens der biometrischen Daten dem Nutzer ein Schall aus **dem Hörgerät** dargeboten wird.

3. Verfahren nach Anspruch 1 oder 2, wobei als biometrische Daten der Hautwiderstand, die Temperatur, der Blutzucker, der Blutsauerstoffgehalt und/oder der Puls des Nutzers gemessen wird.

4. Verfahren nach einem der vorhergehenden Ansprüche, wobei in Abhängigkeit der biometrischen Daten ein Frequenzgang, die Lautstärke (V) und/oder eine Störgeräuschunterdrückung (SU) **des Hörgeräts** angepasst werden.

5. Verfahren nach einem der vorhergehenden Ansprüche, wobei in Abhängigkeit des Pulses des Nutzers ein Rhythmus eines dargebotenen oder darzubietenden Musikstücks angepasst wird.

6. Verfahren nach einem der vorhergehenden Ansprüche, wobei in Abhängigkeit der biometrischen Daten in **dem Hörgerät** mindestens ein Steuersignal erzeugt und an ein **von dem Hörgerät verschiedenes** Gerät gesendet wird.

7. Hörgerät, mit
- einem Mikrofon (M),
- einer Signalverarbeitungseinrichtung (SU, V) zum Verarbeiten eines von dem Mikrofon aufgenommenen Eingangsschals zu einem Ausgangsschall und
- einer Sensoreinrichtung (WM, ME) zum Erfassen biometrischer Daten des Nutzers des Hörgeräts,
**gekennzeichnet durch**
- eine Einstelleinrichtung, an die die Sensoreinrichtung (WM, ME) angeschlossen ist, und die mit der Verarbeitungseinrichtung derart wirkverbunden ist, dass mindestens ein Parameter der Signalverarbeitungseinrichtung (SU, V) automatisch in Abhängigkeit von den erfassten biometrischen Daten eingestellt wird.

8. Hörgerät nach Anspruch 7, die über zwei Abschnitte aus leitfähigem Kunststoff (ME) zur Vermessung des Hautwiderstands (WM) verfügt.

9. Hörgerät nach Anspruch 7 oder 8, die eine Steuereinrichtung zur Steuerung eines von dem Hörgerät verschiedenen Geräts aufweist, wobei die Steuereinrichtung von der Einstelleinrichtung automatisch einstellbar ist.

## Claims

1. Method for adjusting a hearing device which processes an input sound recorded by a microphone (M) of the hearing device to form an output sound,
**characterised by**
- acquiring biometric data (WM) relating to the user of the hearing device and
- adjusting at least one parameter (SU, V) of the hearing device so that the processing of the input sound by means of the hearing device takes place as a function of the acquired biometric data.

2. Method according to claim 1, with a sound from the hearing device being presented to the user immediately before or during the acquisition of biometric data.

3. Method according to claim 1 or 2, with the skin resistance, the temperature, the blood sugar level and the blood oxygen content and/or the pulse of the user being measured as biometric data.

4. Method according to one of the preceding claims, with a frequency response, the volume (V) and/or a noise suppression (SU) of the hearing device being adjusted as a function of the biometric data.

5. Method according to one of the preceding claims, with a rhythm of a presented piece of music or a piece of music to be presented being adjusted as a function of the pulse of the user.

6. Method according to one of the preceding claims, with at least one control signal being generated in the hearing device as a function of the biometric data and being transmitted to a device which differs from the hearing device.

7. Hearing device, having
- a microphone (M),
- a signal processing facility (SU, V) for processing an input sound recorded by the microphone to form an output sound, and
- a sensor facility (WM, ME) for acquiring biometric data relating to the user of the hearing device,
**characterised by**
- an adjustment facility, to which the sensor facility (WM, ME) is connected, and which is actively connected to the processing facility such that at least one parameter of the signal processing facility (SU, V) is automatically adjusted as a function of the acquired biometric data.

8. Hearing device according to claim 7, which has two segments made of conductive plastic (ME) for measuring the skin resistance (WM).

9. Hearing device according to claim 7 or 8, comprising a control facility for controlling a device which differs from the hearing device, with the control facility being automatically adjustable by means of the adjustment facility.

## Revendications

1. Procédé pour régler un appareil auditif, qui transforme un son d'entrée réceptionné par un microphone (M) de l'appareil auditif en un son de sortie,
**caractérisé par**
- la saisie de données biométriques (WM) de l'utilisateur de l'appareil auditif et
- le réglage d'au moins un paramètre (SU, V) de l'appareil auditif, de sorte que le traitement du bruit d'entrée par l'appareil auditif s'effectue en fonction des données biométriques saisies.

2. Procédé selon la revendication 1, un son provenant de l'appareil auditif étant présenté à l'utilisateur juste avant ou pendant la saisie des données biométriques.

3. Procédé selon la revendication 1 ou 2, la résistance de la peau, la température, la glycémie, la teneur en oxygène dans le sang et/ou le pouls de l'utilisateur étant mesurés comme données biométriques.

4. Procédé selon l'une quelconque des revendications précédentes, une courbe de fréquence, l'intensité sonore (V) et/ou une suppression du bruit parasite (SU) de l'appareil auditif étant adaptée(s) en fonction des données biométriques.

5. Procédé selon l'une quelconque des revendications précédentes, un rythme d'un morceau de musique présenté ou à présenter étant adapté en fonction du pouls de l'utilisateur.

6. Procédé selon l'une quelconque des revendications précédentes, au moins un signal de commande étant généré en fonction des données biométriques dans l'appareil auditif et étant envoyé à un appareil différent de l'appareil auditif.

7. Appareil auditif comprenant
- un microphone (M),
- un dispositif de traitement de signal (SU, V) pour le traitement d'un son d'entrée réceptionné par le microphone en un son de sortie et
- un dispositif capteur (WM, ME) pour l'enregistrement de données biométriques de l'utilisateur de l'appareil auditif,
**caractérisée par**
- un dispositif de réglage, auquel le dispositif capteur (WM, ME) est raccordé, et qui est en liaison active avec le dispositif de traitement, de telle sorte qu'au moins un paramètre du dispositif de traitement de signal (SU, V) est réglé automatiquement en fonction des données biométriques enregistrées.

8. Appareil auditif selon la revendication 7, qui dispose de deux parties à base de plastique conducteur (ME) pour la mesure de la résistance de la peau (WM).

9. Appareil auditif selon la revendication 7 ou 8, qui présente un dispositif de commande pour la commande d'un appareil différent de l'appareil auditif, le dispositif de commande pouvant être réglé automatiquement par le dispositif de réglage.
